# EUROPÄISCHE PATENTSCHRIFT

(11) **EP 1 722 734 B1**
(45) Veröffentlichungstag und Bekanntmachung des Hinweises auf die Patenterteilung: **23.05.2007**
(21) Anmeldenummer: 05707461.9
(22) Anmeldetag: 17.02.2005
(51) Int. Cl.: A61F 13/04

(54) **FIXIERMITTEL ZUM TEMPORÄREN FIXIEREN VON MENSCHLICHEN ODER TIERISCHEN KÖRPERTEILEN**
FIXING MEANS FOR TEMPORARILY FIXING HUMAN OR ANIMAL BODY PARTS
MOYEN D'ATTACHE DESTINE A LA FIXATION TEMPORAIRE DE PARTIES DU CORPS D'UN ETRE HUMAIN OU D'UN ANIMAL

(30) Priorität: 11.03.2004 DE 102004011872
(43) Veröffentlichungstag der Anmeldung: 22.11.2006
(73) Patentinhaber: Fraunhofer-Gesellschaft zur Förderung der angewandten Forschung e.V., 80686 München (DE)
(72) Erfinder: ELSNER, Peter, 73271 Holzmaden (DE); GEISSLER, Adam, 76689 Karlsdorf-Neuthard (DE); KNÖRLEIN, Martin, 79102 Freiburg (DE)
(86) Internationale Anmeldenummer: PCT/EP2005/001617
(87) Internationale Veröffentlichungsnummer: WO 2005/087160

(56) Entgegenhaltungen:
- EP-A- 0 305 175
- WO-A-02/13735
- US-A1- 2002 123 709
- US-B1- 6 544 204

## Beschreibung

Die Erfindung betrifft ein Fixiermittel zum temporären Fixieren von menschlichen oder tierischen Körperteilen, mit wenigstens einer flexiblen Trägerschicht, welche mit wenigstens einem aushärtbaren Material beschichtet ist, wobei das zu fixierende Körperteil mit dem Fixiermittel ganz oder teilweise umhüllbar und das aushärtbare Material sodann unter Versteifung der Trägerschicht aushärtbar ist.

In vielen Fällen besteht sowohl auf dem Gebiet der Humanals auch der Veterinärmedizin aus therapeutischer Sicht das Erfordernis einer temporären Fixierung von Körperteilen. Dies gilt beispielsweise im Falle von Knochen-, Knorpel-, Gelenk-, Kapsel-, Sehnenverletzungen oder dergleichen, aber auch bei der Nachbehandlung von operativen Eingriffen, bei Entzündungen, wie Knochenhaut-, Sehnenscheidenentzündungen etc., sowie bei zahlreichen anderen Verletzungen bzw. Erkrankungen.

Gewöhnlich werden als Fixiermittel Gipskompressen aus einem textilen Trägermaterial eingesetzt, welches mit Gipspartikeln beschichtet ist. Die Kompressen werden befeuchtet und um das Körperteil, welches ruhiggestellt werden soll, teilweise oder gänzlich herumgewickelt und unter Abbinden des Gipses zu einem starren Gipsverband ausgehärtet. Alternativ wird der Gips getrennt in Wasser angerührt, das textile Trägermaterial mit der Dispersion getränkt und der Verband nach Umhüllen des Körperteils ausgehärtet.

Abgesehen von dem verhältnismäßig hohen Gewicht solcher Gipsverbände ist insbesondere nachteilig, daß das Gipsmaterial nach dem Abbinden gänzlich starr ist, was insbesondere dann, wenn aus medizinischer Sicht eine verhältnismäßig lange Fixierung von Körperteilen erforderlich ist, schwerwiegende Folgen für den Patienten haben kann. So kommt es insbesondere bei einer längeren Ruhigstellung von Gliedma-ßen häufig zu Muskelschwund, Sehnenverhärtungen, Gelenkstarre und dergleichen mehr, so daß der Patient in vielen Fällen nach Abnahme des Gipsverbandes unter Beschwerden leidet und häufig gar physiotherapeutische Maßnahmen erforderlich sind.

Entsprechendes gilt für in neuerer Zeit gebräuchliche Kunststoffkompressen, welche zwar ein gegenüber Gips geringeres Gewicht aufweisen, aber gleichfalls gänzlich starr sind.

Andererseits sind elastische Stützverbände bekannt, welche allein oder in Verbindung mit einer Fixiereinrichtung, wie beispielsweise einer Schiene, eingesetzt werden. Indes reicht die Fixierwirkung solcher Stützverbände bei vielen medizinischen Indikationen - zumindest im akuten Zustand - nicht aus.

Die DE 198 41 562 C2 beschreibt ein Fixiermittel zum temporären Fixieren von menschlichen oder tierischen Körperteilen in Form eines synthetischen Steifverbandes. Dieser umfaßt ein mit einem Kunstharz getränktes Textil, ein darunter angeordnetes Polster, eine darüber angeordnete Folie sowie einen zwischen dem Textil und der darüber angeordneten Folie eingebrachten Abstandhalter. Nachteilig ist einerseits die äußerst aufwendige Handhabung des bekannten Fixiermittels, weil das mit Kunstharz getränkte Textil in einer feuchtigkeitsdichten Verpackung aufbewahrt werden muß, damit das Kunstharz nicht vorzeitig aushärtet und der Verband unbrauchbar wird. Nach Entnehmen des getränkten Textils müssen dann die weiteren Schichten mit diesem verbunden werden und muß der so zusammengefügte Verband unter Zwischenlage des Polsters dem Patienten angelegt werden. Hierbei muß wiederum sorgfältig darauf geachtet werden, daß das Kunstharz nicht mit der - häufig verletzten - Haut des Patienten in Berührung tritt und dort aushärtet.

Der EP 0 352 095 B1 ist ebenfalls ein orthopädisches Fixiermittel entnehmbar, welches ein flexibles Substrat, insbesondere aus Gewebe- oder Vliesstoff, umfaßt, welches an beiden Oberflächen mit einer härtbaren flüssigen Verbindung imprägniert ist. An seinen beiden Oberflächen ist das imprägnierte Substrat mit einer für die flüssige Verbindung undurchlässigen, aber wasserdurchlässigen Abdeckung bedeckt. Die Aushärtung der härtbaren flüssigen Verbindung geschieht ausschließlich mit Wasser, was zur Folge hat, daß eine nur bereichsweise Aushärtung oder eine bereichsweise Variation der Steifigkeit des ausgehärteten Fixiermittels, wie sei aus medizinischer Sicht und/oder aus Gründen eines für den Patienten höheren Komforts erwünscht sein kann, - wenn überhaupt - nur bedingt möglich ist.

Die WO 02/13735 A2 beschreibt eine härtbare, klebende Schiene, welche ein härtbares Harz umfaßt, wobei unter anderem mittels Wärme, elektromagnetischer Strahlung, UV- und IR-Strahlung aushärtbare Harze vorgesehen sein können. Die aus mehreren Komponenten aufgebaute Schiene wird, nachdem sie von dem Benutzer zusammengesetzt worden ist, unmittelbar auf die Haut des Patienten aufgeklebt.

Aus der DE 199 62 747 A1 ist ein weiteres Fixiermittel in Form einer orthopädischen Bandage bekannt, welches ein flexibles Trägermaterial, insbesondere in Textilform, und eine thermoplastische Klebemasse aufweist. Letztere wird vor der Benutzung durch Erwärmung in einen plastischen Zustand überführt und entsprechend der Kontur des zu fixierenden Körperglieds erstarrt. Auch in diesem Fall ist aufgrund der Vorwärmung eine mehr oder minder zeitaufwendige Vorbehandlung erforderlich und ist eine gezielte bereichsweise Aushärtung praktisch unmöglich.

Der Erfindung liegt die Aufgabe zugrunde, ein einfaches und kostengünstiges Fixiermittel zum temporären Fixieren von menschlichen oder tierischen Körperteilen unter Vermeidung der vorgenannten Nachteile dahingehend weiterzubilden, daß seine Steifigkeit gemäß den individuellen Erfordernissen bereichsweise variierbar ist.

Erfindungsgemäß wird diese Aufgabe bei einem Fixiermittel der eingangs genannten Art dadurch gelöst, daß das Fixiermittel wenigstens zwei Trägerschichten aus zumindest einer äußeren Lage aus einer flexiblen Polymerfolie und zumindest einer inneren Lage aus einer flexiblen Polymerfolie sowie wenigstens eine zwischen der äußeren und der inneren Lage angeordnete, im wesentlichen flüssige oder viskose Schicht aus wenigstens einem aushärtbaren Kleber aufweist.

Das erfindungsgemäße Fixiermittel ermöglicht auf einfache Weise eine individuelle, bereichsweise Einstellung der gewünschten, aus medizinischer bzw. therapeutischer Sicht gebotenen Steifigkeit, indem nach Anlegen des Fixiermittels an das ruhigzustellende Körperteil - sei es unter gänzlicher oder sei es nur unter teilweiser Umhüllung desselben - durch Einwirken eines Druckes auf das Fixiermittel der im wesentlichen flüssige oder viskose Kleber bereichsweise verdrängt bzw. angereichert wird. Dies kann z.B. manuell, beispielsweise durch Zusammendrücken der beiden Polymerfolien gegeneinander bzw. durch Ausüben eines Andruckes gegen die äußere Polymerfolie, durch Knicken des Fixiermittels etc., oder durch beliebige Hilfsmittel, wie Walzen, Rollen oder dergleichen, geschehen. Sodann kann die zwischen der äußeren und der inneren, flexiblen Polymerfolie eingebrachte Kleberschicht ausgehärtet werden, wobei das Fixiermittel in denjenigen Bereichen, in welchen die Kleberschicht ganz oder teilweise verdrängt worden ist, eine gegenüber den übrigen, insbesondere gänzlich starren Bereichen verhältnismäßig hohe Nachgiebigkeit aufweist. Ist das Klebematerial z.B. im wesentlichen vollständig verdrängt worden, so ergibt sich die maximal mögliche Nachgiebigkeit, welche im wesentlichen der Nachgiebigkeit der Lagen aus flexibler Polymerfolie entspricht. Demgegenüber lassen sich, wie bereits angedeutet, die übrigen Bereiche, des Fixiermittels gänzlich starr ausbilden, indem die dort vorhandene Klebeschicht gänzlich ausgehärtet wird. Das erfindungsgemäße Fixiermittel ist dabei bereits fertig verwendbar und muß nicht mehr aus mehreren separaten Komponenten zusammengesetzt werden, wobei die zwischen den Folienlagen angeordnete Kleberschicht zuverlässig davor bewahrt ist, mit der Haut des Patienten in Kontakt zu treten.

Selbstverständlich lassen sich Bereiche mit unterschiedlicher Steifigkeit alternativ oder zusätzlich auch dadurch ausbilden, indem die Klebeschicht bereichsweise gänzlich und in anderen Bereichen nur teilweise oder gar nicht ausgehärtet wird, wie es weiter unten im einzelnen erläutert wird.

Das erfindungsgemäße Fixiermittel, welches im übrigen ein insbesondere gegenüber Gips erheblich geringeres Gewicht aufweist, ist somit hinsichtlich seiner Fixierwirkung exakt an die individuellen Erfordernisse anpaßbar und trägt zur Verhinderung oder jedenfalls zu einer Linderung von Beschwerden des Patienten nach längerer Ruhigstellung eines seiner Körperteile, wie Muskelschwund, Sehnenverhärtungen, Gelenksteifigkeit etc., bei. So erlaubt das erfindungsgemäße Fixiermittel z.B. bei Indikationen, bei welchen eine Fixierung zumindest der beiden an ein verletztes oder erkranktes Glied, z.B. des Fingers, der Hand, des Unterarms etc., angrenzenden Gelenke, z.B. der Fingergelenke, des Handgelenks, des Ellbogens etc., erforderlich ist, auch eine Unterstützung weiterer, benachbarter Gelenke, z.B. des Handgelenks, des Ellbogens, der Schulter etc., ohne letztere gänzlich ruhigstellen zu müssen.

Darüber hinaus lassen sich die in Form von Polymerfolienlagen ausgebildeten Trägerschichten des erfindungsgemäßen Fixiermittels, wie weiter unten noch näher erläutert, sowie auch der zwischen diese eingebrachte Kleber transparent ausgestalten, so daß der Heilungsprozeß eines verletzten oder erkrankten Körpergliedes durch das Fixiermittel hindurch sichtbar ist und letzteres zur Beobachtung des Heilungsprozesses nicht notwendigerweise entfernt und durch ein neues Fixiermittel ersetzt werden muß.

Während es in den meisten Anwendungsfällen ausreicht, wenn das erfindungsgemäße Fixiermittel nur jeweils eine innere und eine äußere Lage aus einer nachgiebigen Polymerfolie mit einer dazwischen angeordneten Kleberschicht aufweist, können selbstverständlich auch mehrere Lagen aus Polymerfolie vorgesehen sein, wobei zwischen wenigstens zwei, mehrere oder auch sämtliche Polymerfolien eines solchen, sandwichartigen Aufbaus die Schicht aus einem aushärtbaren Kleber eingebracht ist.

Eine bevorzugte Ausführung sieht vor, daß der Kleber ein mittels elektromagnetischer Strahlung aushärtbarer Kleber ist und zumindest die äußere Lage aus einer für elektromagnetischen Strahlung durchlässigen Polymerfolie besteht. Die Aushärtung des Kleber geschieht in diesem Fall beispielsweise mittels einer elektromagnetischen Strahlungsquelle, wobei eine gezielte Bestrahlung derjenigen Bereiche des Fixiermittels möglich ist, welche gänzlich ausgehärtet werden sollen, während andere Bereiche, welche nur teilweise bzw. gar nicht ausgehärtet werden sollen, über einen demgegenüber kürzeren Zeitraum und oder mit einer demgegenüber geringeren Intensität der elektromagnetischen Strahlung bzw. gar nicht bestrahlt werden können.

Der Kleber kann beispielsweise ein mittels Infrarot-Strahlung aushärtbarer Kleber sein, wobei mit Infrarot-Strahlung elektromagnetische Strahlung mit einer Wellenlänge von etwa 800 nm bis etwa 1000 µm, also oberhalb des sichtbaren Lichtes, angesprochen ist.

Alternativ kann der Kleber beispielsweise ein mittels Ultraviolett-Strahlung aushärtbarer Kleber sein, wobei mit Ultraviolett-Strahlung elektromagnetische Strahlung mit einer Wellenlänge von etwa 10 nm bis 400 nm, also unterhalb des sichtbaren Lichtes, aber oberhalb von Röntgen-Stahlung, angesprochen ist. Um den Körper des Patienten soweit möglich zu schonen, ist dabei ist vorzugsweise ein Kleber vorgesehen, welcher mittels langwelliger und somit relativ energiearmer Ultraviolett-Strahlung aushärtbar ist, vorzugsweise in einem Wellenlängenbereich von UV-A-Strahlung (ca. 320 nm bis ca. 400 nm) über UV-B-Strahlung (ca. 280 nm bis ca. 320 nm) bis hin zu UV-C-Strahlung (ca. 200 nm bis ca. 280 nm), insbesondere im UV-A- bis UV-B-Bereich, höchst vorzugsweise im UV-A-Bereich.

Eine andere bevorzugte Ausführung sieht vor, daß der Kleber ein mittels Ultraschall aushärtbarer Kleber ist, wobei auch in diesem Fall die Möglichkeit einer nur bereichsweisen Aushärtung des Fixiermittels gegeben ist, indem das Fixiermittel mittels eines Ultraschallsenders, wie einer Sonotrode, bereichsweise über einen unterschiedlichen Zeitraum und/oder mit unterschiedlicher Intensität beschallt wird.

Schließlich sieht eine weitere bevorzugte Ausführung vor, daß der Kleber ein mittels Wärme aushärtbarer Kleber ist. Dabei kann der Kleber einerseits mittels Körperwärme aushärtbar sein, sofern eine gänzliche Aushärtung erwünscht ist und gegebenenfalls die Ausbildung unterschiedlicher Steifigkeiten durch bereichsweises Verdrängen der Kleberschicht geschehen soll, wie es vorstehend erläutert ist. Andererseits kann der Kleber auch so gewählt werden, daß zur Aushärtung eine gegenüber der Körperwärme erhöhte Wärme erforderlich ist, beispielsweise im Bereich zwischen etwa 40 und etwa 60°C, insbesondere zwischen etwa 40°C und etwa 50°C, so daß sich dem Fixiermittel auch in diesem Fall durch bereichsweises Erwärmen unter Aushärten des Klebers unterschiedliche Steifigkeiten verleihen lassen, ohne daß die Gefahr einer Nachhärtung infolge Körperwärme besteht.

Der Kleber kann vorzugsweise von wenigstens einem aushärtbaren Harz, gegebenenfalls in Verbindung mit einem mittels elektromagnetischer Strahlung, Ultraschall und/oder Wärme aktivierbaren Härter, gebildet sein. Als derartige Harzsysteme kommen insbesondere Duroplaste in Betracht, vorzugsweise aus der Gruppe Acrylharze, Methacrylharze, Epoxidharze, Polyesterharze, Polyurethanharze, Harnstoffharze, Melaninharze, Formaldehydharze, Phenolharze, Furanharze, Silikonharze, Mischungen hiervon oder einem durch Copolymerisation mehrerer der genannten Harze erhaltenen Duroplast.

Die äußere und/oder die innere Lage des erfindungsgemäßen Fixiermittels ist/sind vorzugsweise von einer Folie aus wenigstens einem thermoplastischen und/oder thermoelastischen Polymer bzw. aus einem Polymer-Blend solcher Polymere gebildet. Derartige Thermoplastfolien weisen eine hinreichende Flexibilität auf, um sie auf einfache Weise nach Art eines Verbandes bzw. einer Kompresse manuell an das ruhigzustellende Körperteil anzulegen. Sie sind ferner auf einfache und kostengünstige Weise mittels beliebiger bekannter thermoplastischer Herstellungsverfahren, z.B. durch Blasformen, Extrudieren und dergleichen, herstellbar. Eine sehr einfache und kostengünstige Herstellung des erfindungsgemäßen Fixiermittels kann in diesem Fall z.B. dadurch geschehen, indem die beiden Folienlagen aus einem thermoplastischen bzw. thermoelastischen Polymer zwischen zwei Zylinderwalzen geführt und der Kleber zwischen die beiden Lagen eingebracht und mittels der Walzen gleichmäßig verteilt wird.

In bevorzugter Ausführung ist vorgesehen, daß die äußere und/oder die innere Lage von einer Folie aus wenigstens einem thermoplastischen und/oder thermoelastischen Polymer mit funktionellen Gruppen, wie Polyamide, Polyester, Polyurethane, Polycarbonate oder dergleichen, gebildet ist/sind. Die funktionellen Gruppen des/der Polymers/Polymere der Folienlagen sorgen für eine einwandfreie und dauerhafte Verbindung mit einer zwischen der äußeren und der inneren Lage eingebrachten duroplastischen Harzschicht, wobei die funktionellen Gruppen der Polymere der äußeren und der inneren Lage anläßlich der Aushärtung der duroplastischen Kleberschicht gegebenenfalls mit dieser vernetzt werden können.

Die Schichtdicke der Lagen der flexiblen Polymerfolien bzw. der Kleberschicht richtet sich vornehmlich nach den jeweils zu fixierenden Körperteilen, wobei in den meisten Fällen eine Schichtdicke zwischen etwa 0,01 mm und etwa 5 mm, insbesondere zwischen etwa 0,1 mm und etwa 2 mm, z.B. bis etwa 0,2 mm, sowohl für die äußere als auch für die innere Lage günstig ist. Entsprechendes gilt hinsichtlich der Kleberschicht für eine Schichtdicke zwischen etwa 0,01 mm und etwa 20 mm, insbesondere zwischen etwa 0,1 mm und etwa 10 mm.

In Weiterbildung kann vorgesehen sein, daß zumindest die Kleberschicht Verstärkungsfasern, insbesondere Glasfasern, aufweist, um im Falle einer gänzlichen Aushärtung des Klebers für eine besonders hohe Festigkeit bzw. Steifigkeit zu sorgen des Fixiermittels zu sorgen.

In vorteilhafter Ausgestaltung ist vorgesehen, daß die Lagen aus flexibler Polymerfolie sowie die Kleberschicht transparent sind, so daß das fixierte Körperteil unter dem Fixiermittel sichtbar bleibt. Eine solche Ausgestaltung bietet sich insbesondere im Falle von Verletzungen an, um den Heilungsprozeß optisch verfolgen zu können und beispielsweise eine Entzündung schnell erkennbar zu machen, ohne das Fixiermittel abnehmen zu müssen.

Das erfindungsgemäße Fixiermittel kann grundsätzlich eine praktisch beliebige, im wesentlichen ebene Form aufweisen. So kann es beispielsweise etwa rechteckförmig ausgebildet sein, sofern das zu fixierende Körperteil nur teilweise umhüllt und das Fixiermittel unter Bildung einer einseitig offenen Schiene ausgehärtet werden soll. Ferner kann es von Vorteil sein, wenn das erfindungsgemäße Fixiermittel etwa rohrförmig nach Art einer Manschette ausgebildet ist, welche z.B. über eine zu fixierende Gliedmaße des Patienten übergeschoben, manuell an die Gliedmaße angedrückt und die zwischen der inneren und der äußeren, jeweils rohrförmigen und koaxial angeordneten Lage eingebrachte Kleberschicht anschließend ausgehärtet wird.

In vielen Fällen ist es indes insbesondere aus handhabungstechnischen Gründen günstig, wenn das Fixiermittel im wesentlichen bandförmig ist, so daß der behandelnde Arzt - sei es ein Humanmediziner oder ein Veterinär - das Fixiermittel nach Art eines Verbandes auf das ruhigzustellende Körperteil aufformen und sodann bereichsweise oder auch gänzlich aushärten kann.

Im letztgenannten Fall kann das bandförmige Fixiermittel insbesondere im wesentlichen rollenförmig gewickelt sein.

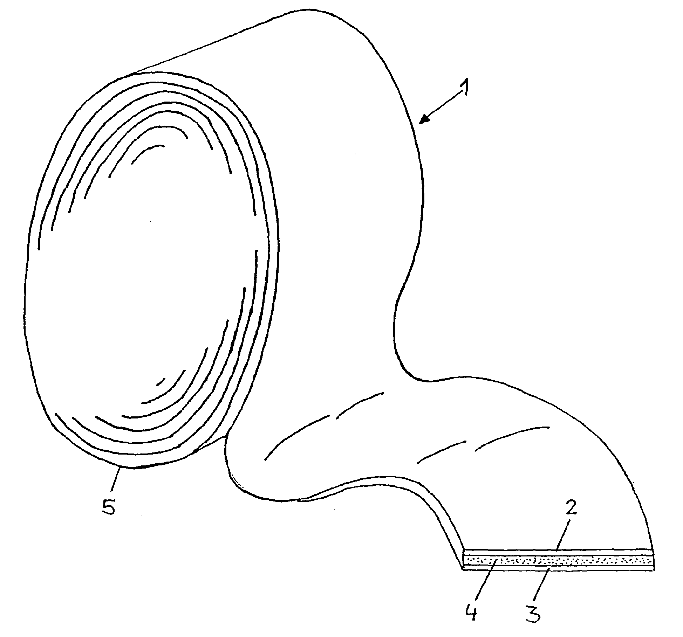

Nachfolgend ist die Erfindung anhand eines Ausführungsbeispiels unter Bezugnahme auf die Zeichnung näher erläutert. Dabei zeigt die einzelne Figur:
eine Ausführungsform eines Fixiermittels zum temporären Fixieren von menschlichen oder tierischen Körperteilen in Form eines bandförmigen Verbandmaterials.

Das in der Zeichnung dargestellte Fixiermittel 1 ist nach Art eines Verbandes ausgebildet und etwa rollenförmig gewickelt. Das bandförmige Fixiermittel 1 umfaßt eine äußere Lage 2 und eine innere Lage 3, welche jeweils aus einer nachgiebigen Kunststoffolie aus einem thermoplastischen Polymer, beispielsweise aus Polyamid, gebildet sind. Die Schichtdicke der äußeren 2 sowie der inneren Lage 3 beträgt beispielsweise etwa 0,06 mm. Zwischen der äußeren 2 und der inneren Lage 3 ist eine Schicht 4 aus einem aushärtbaren Kleber in Form einer viskosen, duroplastischen Harzmischung eingebracht, deren Schichtdicke beispielsweise etwa 5 mm betragen kann.

Die Harzmischung kann z.B. aus 70 Mass.-% iso-Butylmethacrylat, 20 Mass.-% 1,3-Butandioldimethacrylat und etwa 10 Mass.-% Trimethylolpropantrimethacrylat bestehen und etwa 1 bis 3 Mass.-% (bezogen auf die Harzmischung) eines photochemischen Härters in Form von Diphenyl(2,4,6-trimethylbenzoyl)phosphinoxid (Handelsname: Lucirin TPO^{®} der BASF AG) versetzt sein.

Das bandförmige Fixiermittel 1 ist beim vorliegenden Ausführungsbeispiel zu einer Rolle 5 gewickelt, so daß es etwa die Form einer Mullbinde aufweist und auf einfache Weise um das zu fixierende Körperteil herumwickelbar ist. Die Lagen 2, 3 sowie die Kleberschicht 4 ist im wesentlichen durchsichtig, wobei die Kleberschicht 4 zum Zwecke der Verstärkung gegebenenfalls mit Mikrofasern, beispielsweise Glasfasern, versetzt sein kann.

## Patentansprüche

1. Fixiermittel zum temporären Fixieren von menschlichen oder tierischen Körperteilen, mit wenigstens einer flexiblen Trägerschicht, welche mit wenigstens einem aushärtbaren Material beschichtet ist, wobei das zu fixierende Körperteil mit dem Fixiermittel ganz oder teilweise umhüllbar und das aushärtbare Material sodann unter Versteifung der Trägerschicht aushärtbar ist, wobei das Fixiermittel (1) wenigstens zwei Trägerschichten aus zumindest einer äußeren Lage (2) aus einer flexiblen Polymerfolie und zumindest einer inneren Lage (3) aus einer flexiblen Polymerfolie aufweist, **dadurch gekennzeichnet, daß** das Fixiermittel (1) wenigstens eine zwischen der äußeren (2) und der inneren Lage (3) angeordnete, im wesentlichen flüssige oder viskose Schicht (4) aus wenigstens einem aushärtbaren Kleber aufweist, wobei die Lagen (2, 3) aus flexibler Polymerfolie sowie die Kleberschicht (4) transparent sind, so daß das fixierte Körperteil unter dem Fixiermittel (1) sichtbar bleibt.

2. Fixiermittel nach Anspruch 1, **dadurch gekennzeichnet, daß** der Kleber ein mittels elektromagnetischer Strahlung aushärtbarer Kleber ist und zumindest die äußere Lage (2) aus einer für elektromagnetische Strahlung durchlässigen Polymerfolie besteht.

3. Fixiermittel nach Anspruch 2, **dadurch gekennzeichnet, daß** der Kleber ein mittels Infrarot-Strahlung aushärtbarer Kleber ist.

4. Fixiermittel nach Anspruch 2, **dadurch gekennzeichnet, daß** der Kleber ein mittels Ultraviolett-Strahlung aushärtbarer Kleber ist.

5. Fixiermittel nach Anspruch 1, **dadurch gekennzeichnet, daß** der Kleber ein mittels Ultraschall aushärtbarer Kleber ist.

6. Fixiermittel nach Anspruch 1, **dadurch gekennzeichnet, daß** der Kleber ein mittels Wärme aushärtbarer Kleber ist.

7. Fixiermittel nach einem der Ansprüche 1 bis 6, **dadurch gekennzeichnet, daß** der Kleber von wenigstens einem aushärtbaren Harz, gegebenenfalls in Verbindung mit einem mittels elektromagnetischer Strahlung, Ultraschall und/oder Wärme aktivierbaren Härter, gebildet ist.

8. Fixiermittel nach Anspruch 7, **dadurch gekennzeichnet, daß** der Kleber von einem Duroplast, insbesondere aus der Gruppe Acrylharze, Methacrylharze, Epoxidharze, Polyesterharze, Polyurethanharze, Harnstoffharze, Melaninharze, Formaldehydharze, Phenolharze, Furanharze, Silikonharze, Mischungen hiervon oder einem durch Copolymerisation mehrerer der genannten Harze erhaltenen Duroplast, gebildet ist.

9. Fixiermittel nach einem der Ansprüche 1 bis 8, **dadurch gekennzeichnet, daß** die äußere (2) und/oder die innere Lage (3) von einer Folie aus wenigstens einem thermoplastischen und/oder thermoelastischen Polymer gebildet ist/sind.

10. Fixiermittel nach Anspruch 9, **dadurch gekennzeichnet, daß** die äußere (2) und/oder die innere Lage (3) von einer Folie aus wenigstens einem thermoplastischen und/oder thermoelastischen Polymer mit funktionellen Gruppen, wie Polyamide, Polyester, Polyurethane, Polycarbonate oder dergleichen, gebildet ist/sind.

11. Fixiermittel nach einem der Ansprüche 1 bis 10, **dadurch gekennzeichnet, daß** die äußere (2) und die innere Lage (3) eine Schichtdicke zwischen 0,01 mm und 5 mm, insbesondere zwischen 0,1 mm und 2 mm aufweist.

12. Fixiermittel nach einem der Ansprüche 1 bis 11, **dadurch gekennzeichnet, daß** die Kleberschicht (4) eine Schichtdicke zwischen 0,01 mm und 20 mm, insbesondere zwischen 0,1 mm und 10 mm aufweist.

13. Fixiermittel nach einem der Ansprüche 1 bis 12, **dadurch gekennzeichnet, daß** zumindest die Kleberschicht (4) Verstärkungsfasern, insbesondere Glasfasern, aufweist.

14. Fixiermittel nach einem der Ansprüche 1 bis 13, **dadurch gekennzeichnet, daß** es im wesentlichen bandförmig ist.

15. Fixiermittel nach Anspruch 14, **dadurch gekennzeichnet, daß** es im wesentlichen rollenförmig gewickelt ist.

## Claims

1. Fixing means for temporarily fixing human or animal body parts, having at least one flexible carrier layer which is coated with at least one curable material, the body part to be fixed being able to be covered entirely or partially with the fixing means and the curable material then being able to be cured with stiffening of the carrier layer, the fixing means (1) having at least two carrier layers comprising at least one outer layer (2) made of a flexible polymer film and at least one inner layer (3) made of a flexible polymer film, **characterised in that** the fixing means (1) has at least one substantially liquid or viscous layer (4) which is made of at least one curable adhesive and is disposed between the outer (2) and the inner layer (3), the layers (2, 3) made of flexible polymer film and also the adhesive layer (4) being transparent so that the fixed body part remains visible under the fixing means (1).

2. Fixing means according to claim 1, **characterised in that** the adhesive is an adhesive which can be cured by means of electromagnetic radiation and at least the outer layer (2) comprises a polymer film which is permeable for electromagnetic radiation.

3. Fixing means according to claim 2, **characterised in that** the adhesive is an adhesive which can be cured by means of infrared radiation.

4. Fixing means according to claim 2, **characterised in that** the adhesive is an adhesive which can be cured by means of ultraviolet radiation.

5. Fixing means according to claim 1, **characterised in that** the adhesive is an adhesive which can be cured by means of ultrasound.

6. Fixing means according to claim 1, **characterised in that** the adhesive is an adhesive which can be cured by means of heat.

7. Fixing means according to one of the claims 1 to 6, **characterised in that** the adhesive is formed from at least one curable resin, if necessary in conjunction with a hardener which can be activated by means of electromagnetic radiation, ultrasound and/or heat.

8. Fixing means according to claim 7, **characterised in that** the adhesive is formed by a duroplastic, in particular from the group acrylic resins, methacrylic resins, epoxy resins, polyester resins, polyurethane resins, urea resins, melamine resins, formaldehyde resins, phenol resins, furan resins, silicone resins, mixtures thereof or a duroplastic obtained by copolymerisation of a plurality of the mentioned resins.

9. Fixing means according to one of the claims 1 to 8, **characterised in that** the outer (2) and/or the inner layer (3) is/are formed from a film made of at least one thermoplastic and/or thermoelastic polymer.

10. Fixing means according to claim 9, **characterised in that** the outer (2) and/or the inner layer (3) is/are formed from a film made of at least one thermoplastic and/or thermoelastic polymer with functional groups, such as polyamides, polyesters, polyurethanes, polycarbonates or the like.

11. Fixing means according to one of the claims 1 to 10, **characterised in that** the outer (2) and the inner layer (3) have a layer thickness between 0.01 mm and 5 mm, in particular between 0.1 mm and 2 mm.

12. Fixing means according to one of the claims 1 to 11, **characterised in that** the adhesive layer (4) has a layer thickness between 0.01 1 mm and 20 mm, in particular between 0.1 mm and 10 mm.

13. Fixing means according to one of the claims 1 to 12, **characterised in that** at least the adhesive layer (4) has reinforcing fibres, in particular glass fibres.

14. Fixing means according to one of the claims 1 to 13, **characterised in that** it is essentially strip-shaped.

15. Fixing means according to claim 14, **characterised in that** it is essentially wound up in a roll-shape.

## Revendications

1. Moyen d'attache destiné à la fixation temporaire de parties du corps d'un être humain ou d'un animal, doté d'au moins une couche de base souple recouverte d'au moins une matière durcissable, la partie du corps à fixer pouvant être enveloppée entièrement ou partiellement par le moyen d'attache et la matière durcissable pouvant durcir immédiatement lors de la solidification de la couche de base, le moyen d'attache (1) présentant au moins deux couches de base composées d'au moins une couche extérieure (2) en feuille de polymère souple et d'au moins une couche intérieure (3) en feuille de polymère souple,
**caractérisé en ce que**
le moyen d'attache (1) présente au moins une couche (4) essentiellement liquide ou visqueuse disposée entre la couche extérieure (2) et la couche intérieure (3) et composée d'au moins une colle durcissable, les couches (2, 3) en feuille de polymère souple ainsi que la couche de colle (4) étant transparentes de sorte que la partie du corps fixée reste visible sous le moyen d'attache (1).

2. Moyen d'attache selon la revendication 1,
**caractérisé en ce que**
la colle est une colle durcissable par rayonnement électromagnétique et au moins la couche extérieure (2) est constituée d'une feuille de polymère laissant passer le rayonnement électromagnétique.

3. Moyen d'attache selon la revendication 2,
**caractérisé en ce que**
la colle est une colle durcissable par rayonnement infrarouge.

4. Moyen d'attache selon la revendication 2,
**caractérisé en ce que**
la colle est une colle durcissable par rayonnement ultraviolet.

5. Moyen d'attache selon la revendication 1,
**caractérisé en ce que**
la colle est une colle durcissable par ultrasons.

6. Moyen d'attache selon la revendication 1,
**caractérisé en ce que**
la colle est une colle thermodurcissable.

7. Moyen d'attache selon l'une quelconque des revendications 1 à 6,
**caractérisé en ce que**
la colle est formée par au moins une résine durcissable, le cas échéant associée à un durcisseur pouvant être activé par au moins un rayonnement électromagnétique, des ultrasons et/ou la chaleur.

8. Moyen d'attache selon la revendication 7,
**caractérisé en ce que**
la colle est une résine durcissable, notamment du groupe résines acryliques, résines métacryliques, résines époxy, résine polyesther, résines polyuréthaniques, résines uréiques, résines mélanine, résines formaldéhydes, résines phénoliques, résines furaniques, résines de silicones, mélanges de deux de ces résines ou plus, ou une résine durcissable obtenue par copolymérisation de plusieurs des résines citées.

9. Moyen d'attache selon l'une quelconque des revendications 1 à 8,
**caractérisé en ce que**
la position extérieure (2) et/ou intérieure (3) est formée par une feuille composée d'au moins un polymère thermoplastique et/ ou thermoélastique.

10. Moyen d'attache selon la revendication 9,
**caractérisé en ce que**
la couche extérieure (2) et/ou la couche intérieure (3) est formée par une feuille composée d'au moins un polymère thermoplastique et/ou thermoélastique ayant des groupes fonctionnels comme le polyamide, le polyester, le polyuréthane, le polycarbonate ou similaire.

11. Moyen d'attache selon l'une quelconque des revendications 1 à 10,
**caractérisé en ce que**
la couche extérieure (2) et la couche intérieure (3) présentent une épaisseur comprise entre 0,01 mm et 5 mm, notamment entre 0,1 mm et 2 mm.

12. Moyen d'attache selon l'une quelconque des revendications 1 à 11,
**caractérisé en ce que**
la couche de colle (4) présente une épaisseur comprise entre 0,01 mm et 20 mm, notamment entre 0,1 mm et 10 mm.

13. Moyen d'attache selon l'une quelconque des revendications 1 à 12,
**caractérisé en ce qu'**
au moins la couche de colle (4) présente des fibres renforcées, notamment des fibres de verre.

14. Moyen d'attache selon l'une quelconque des revendications 1 à 13,
**caractérisé en ce qu'**
il est essentiellement en forme de bande.

15. Moyen d'attache selon la revendication 14,
**caractérisé en ce qu'**
il est essentiellement enroulé sous forme de rouleau.
